(19) Europäisches Patentamt
European Patent Office
Office européen des brevets

(11) Veröffentlichungsnummer: **0 477 666 B1**

# (12) EUROPÄISCHE PATENTSCHRIFT

(45) Veröffentlichungstag der Patentschrift: **28.06.95**

(21) Anmeldenummer: **91115368.2**

(22) Anmeldetag: **11.09.91**

(51) Int. Cl.⁶: **C07D 303/16**, C07D 303/40, C07D 303/24, C07D 303/34, G02F 1/35

## (54) Vernetzte Epoxidharze mit nichtlinear-optischen Eigenschaften.

(30) Priorität: **24.09.90 DE 4030179**

(43) Veröffentlichungstag der Anmeldung:
**01.04.92 Patentblatt 92/14**

(45) Bekanntmachung des Hinweises auf die Patenterteilung:
**28.06.95 Patentblatt 95/26**

(84) Benannte Vertragsstaaten:
**CH DE FR GB IT LI NL SE**

(56) Entgegenhaltungen:
**EP-A- 0 231 770**

**GARO KHANARIAN, EDITOR 'Nonlinear Optical Properties of Organic Materials II, Proceedings, 10-11 August 1989, San Diego, California' 1989 , SPIE - THE INTERNATIONAL SOCIETY FOR OPT. ENG. , WASHINGTON, US Iain A McCulloch et al., "Synthesis and Characterisation of Liquid Crystalline Nonlinear Polymers"**

**Journal of Applied Physics, vol. 66, p. 3241-7**

(73) Patentinhaber: **SIEMENS AKTIENGESELLSCHAFT**
**Wittelsbacherplatz 2**
**D-80333 München (DE)**

(72) Erfinder: **Nordmann, Jens, Dr.**
**Sandsteinstrasse 23**
**W-8524 Neunkirchen/Brand (DE)**
Erfinder: **Hacker, Heinz, Dr.**
**Kaiserslauterer Strasse 9**
**W-8500 Nürnberg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft neue Epoxidharze und deren Verwendung.

Die nichtlineare Optik befaßt sich mit der Wechselwirkung des elektromagnetischen Feldes einer sich in einem Medium ausbreitenden Lichtwelle mit diesem Medium sowie mit dem damit verbundenen Entstehen neuer Felder mit veränderten Eigenschaften. Tritt nämlich das elektromagnetische Feld mit dem aus einem Molekül oder aus vielen Molekülen bestehenden Medium in Wechselwirkung, so polarisiert dieses Feld die Moleküle.

Die Polarisation, die durch ein lokales elektrisches Feld in einem Molekül induziert wird, kann - entsprechend Gleichung (1) - als Potenzreihe der elektrischen Feldstärke dargestellt werden:

$$P = \alpha.E + \beta.E^2 + \gamma.E^3 + ... \quad (1);$$

P ist dabei die induzierte Polarisation und E das induzierte lokale elektrische Feld, und $\alpha$, $\beta$ und $\gamma$ stellen die Polarisierbarkeit erster, zweiter und dritter Ordnung dar.

Auf makroskopischer Ebene gilt - entsprechend Gleichung (2) - für die durch ein äußeres elektrisches Feld in einem aus mehreren Molekülen bestehenden Medium induzierte Polarisation eine ähnliche Beziehung:

$$P = \epsilon_o(\chi^{(1)}.E + \chi^{(2)}.E^2 + \chi^{(3)}.E^3 + ...) \quad (2);$$

P ist dabei wiederum die induzierte Polarisation und E das induzierte lokale elektrische Feld $\epsilon_o$ ist die Dielektrizitätskonstante und $\chi^{(1)}$, $\chi^{(2)}$ und $\chi^{(3)}$ stellen die dielektrische Suszeptibilität erster, zweiter und dritter Ordnung dar.

Die dielektrischen Suszeptibilitäten in Gleichung (2) haben eine ähnliche Bedeutung wie die molekularen Koeffizienten in Gleichung (1): Sie sind Materialkonstanten, die von der Molekülstruktur und der Frequenz und im allgemeinen auch von der Temperatur abhängen. Die Koeffizienten $\chi^{(2)}$ und $\chi^{(3)}$ bewirken eine Vielzahl nichtlinear-optischer Effekte, und zwar je nach der Eingangsfrequenz und dem Abstand der molekularen Schwingungsfrequenzen oder elektronischen Resonanzen und den Eingangsfrequenzen oder Frequenzkombinationen sowie den Phasenanpassungsbedingungen.

Materialien mit einer dielektrischen Suszeptibilität zweiter Ordnung eignen sich zur Frequenzverdoppelung (SHG = Second Harmonic Generation); dies ist die Umwandlung von Licht der Frequenz $\omega$ in solches der Frequenz $2\omega$. Ein weiterer nichtlinearoptischer Effekt zweiter Ordnung ist der lineare elektrooptische Effekt (Pockels-Effekt) ; er resultiert aus der Änderung des Brechungsindex des optischen Mediums bei angelegtem elektrischen Feld. Die optische Gleichrichtung sowie die Summen- und Differenzfrequenzmischung sind weitere Beispiele für nichtlinear-optische Effekte zweiter Ordnung.

Einsatzgebiete für Materialien der vorstehend genannten Art sind beispielsweise elektrooptische Schalter sowie Bereiche der Informationsverarbeitung und der integrierten Optik, wie optische chip-to-chip-Verbindungen, wave-guiding in elektrooptischen Schichten, Mach-Zehnder-Interferometer und optische Signalverarbeitung in der Sensorik.

Materialien mit einer dielektrischen Suszeptibilität dritter Ordnung eignen sich zur Frequenzverdreifachung der eingestrahlten Lichtwelle. Weitere Effekte dritter Ordnung sind die optische Bistabilität und die Phasenkonjugation. Konkrete Anwendungsbeispiele sind der rein optische Schalter zum Aufbau rein optischer Computer und die holographische Datenverarbeitung.

Zur Erzielung eines ausreichenden nichtlinear-optischen Effektes zweiter Ordnung muß die dielektrische Suszeptibilität zweiter Ordnung $\chi^{(2)}$ größer sein als $10^{-9}$ elektrostatische Einheiten (esu); dies bedeutet, daß die Hyperpolarisierbarkeit $\beta$ größer sein muß als $10^{-30}$ esu. Eine weitere fundamentale Voraussetzung zur Erzielung eines nichtlinear-optischen Effektes zweiter Ordnung ist die nicht-zentrosymmetrische Orientierung der Moleküle im nichtlinear-optischen Medium; andernfalls wird nämlich $\chi^{(2)} = 0$. Dies kann, wenn nicht - wie bei kristallinen Materialien - durch die Kristallstruktur vorgegeben, durch eine Orientierung der molekularen Dipole erreicht werden. So sind die größten Werte von $\chi^{(2)}$ für ein nichtlinear-optisches Medium durch Orientierung der molekularen Dipole in elektrischen Feldern erreicht worden.

Anorganische Materialien, wie Lithiumniobat ($LiNbO_3$) und Kaliumdihydrogenphosphat ($KH_2PO_4$), zeigen nichtlinear-optische Eigenschaften. Auch Halbleitermaterialien, wie Galliumarsenid (GaAs), Galliumphosphid (GaP) und Indiumantimonid (InSb), weisen nichtlinear-optische Eigenschaften auf.

Neben dem Vorteil eines hohen elektrooptischen Koeffizienten zweiter Ordnung haben anorganische Materialien der genannten Art jedoch einige entscheidende Nachteile. So ist die Verarbeitung dieser Materialien technisch sehr aufwendig, da einzelne Prozeßschritte zeitaufwendig sind und mit höchster

Genauigkeit durchgeführt werden müssen (siehe dazu: C. Flytzanis und J.L. Oudar "Nonlinear Optics: Materials and Devices", Springer-Verlag (1986), Seiten 2 bis 30). Diese Materialien sind ferner für solche elektrooptische Bauteile ungeeignet, die bei hohen Modulationsfrequenzen arbeiten. Bedingt durch die intrinsich vorhandenen hohen Dielektrizitätskonstanten treten bei hohen Frequenzen (oberhalb einiger GHz) nämlich so hohe dielektrische Verluste auf, daß ein Arbeiten bei diesen Frequenzen unmöglich ist (siehe dazu: "J. Opt. Soc. Am. B", Vol. 6 (1989), Seiten 685 bis 692).

Es ist bekannt, daß organische und polymere Materialien mit ausgedehnten $\pi$-Elektronensystemen, die mit Elektronendonatoren und -akzeptoren substituiert sind, nichtlinear-optische Eigenschaften aufweisen, d.h. in nichtlinear-optischen Medien Verwendung finden können (siehe dazu: R.A. Hann und D. Bloor "Organic Materials for Non-linear Optics", The Royal Society of Chemistry (1989), Seiten 382 bis 389 und 404 bis 411).

Einkristalle auf organischer Basis weisen - im Vergleich zu $LiNbO_3$ - einen hohen elektrooptischen Koeffizienten zweiter Ordnung und eine gute photochemische Stabilität auf; auch die erforderliche hohe Orientierung der nichtlinear-optischen Moleküle ist bereits gegeben. Einige wesentliche Kriterien sprechen jedoch gegen eine technische Nutzung dieser Materialklasse. So wird für die Herstellung der Einkristalle, und zwar sowohl aus einer Lösung als auch aus der Schmelze, eine Zeit von 14 bis 30 Tagen benötigt (siehe dazu: D.S. Chemla und J. Zyss "Nonlinear Optical Properties of Organic Molecules and Crystals", Academic Press, Inc. (1987), Vol. 1, Seiten 297 bis 356); der Herstellungsprozeß wird somit einer technischen Fertigung nicht gerecht. Außerdem liegt der Schmelzpunkt der Kristalle im Mittel bei 100 °C, so daß ein Arbeitstemperaturbereich bis 90 °C nicht zu realisieren sein dürfte. Ferner sind organische Kristalle nicht strukturierbar und ihre lateralen Abmessungen sind gegenwärtig noch zu gering, um einen Aufbau als elektrooptisches Bauelement zu ermöglichen.

Für Anwendungen der nichtlinearen Optik in den Bereichen der Informationsübertragung und der integrierten Optik finden in neuerer Zeit polymere Materialien eine zunehmende Bedeutung. Dazu wird an die über die Glasübergangstemperatur erwärmten polymeren Materialien ein äußeres elektrisches Feld angelegt; dies führt zu einer Orientierung der nichtlinear-optischen Moleküle. Nach dem Abkühlen (auf Temperaturen unterhalb der Glasübergangstemperatur), bei angelegtem elektrischen Feld, erhält man anisotrope und somit nicht-zentrosymmetrische Polymere, die dielektrische Suszeptibilitäten zweiter Ordnung aufweisen.

Nichtlinear-optische Verbindungen, die in Polymeren gelöst oder in Polymere eindiffundiert sind, lassen sich in dieser Weise zu dünnen Schichten verarbeiten, wie dies von der integrierten Optik gefordert wird (siehe dazu: "Macromolecules", Vol. 15 (1982), Seiten 1385 bis 1389; "Appl. Phys. Lett.", Vol. 49 (1986), Seiten 248 bis 250; "Electron. Lett.", Vol. 23 (1987), Seiten 700 und 701). Nachteilig wirkt sich hierbei jedoch die geringe Löslichkeit der niedermolekularen Verbindungen, ihre ungenügende Verteilung in den Polymeren, die Migration der aktiven Moleküle aus der Polymermatrix und der Verlust der nichtzentrosymmetrischen Orientierung der aktiven Molekülspezies innerhalb einiger Stunden, schon bei Raumtemperatur, aus.

Als nichtlinear-optische Verbindungen sind auch Polymere mit kovalent gebundenen nichtlinear-optischen Molekülbausteinen bekannt, die gleichzeitig einen flüssigkristallinen Charakter besitzen (siehe dazu: EP-OS 0 231 770 und EP-OS 0 262 680). Diese Materialien weisen zwar die vorstehend genannten Nachteile nicht auf, sie sind aber im gegenwärtigen Entwicklungsstadium für Anwendungen in der Elektro- oder integrierten Optik nicht geeignet, da sich hierbei optische Verluste > 20 dB/cm ergeben, die durch die inhärente Domänenstreuung hervorgerufen werden. Ferner wurde bereits über Untersuchungen an amorphen nichtlinear-optischen Polymeren berichtet (siehe: "Macromolecules", Vol. 21 (1988), Seiten 2899 bis 2901).

Sowohl bei flüssigkristallinen als auch bei amorphen Polymeren mit kovalent gebundenen nichtlinear-optischen Moleküleinheiten kann eine hohe Konzentration an derartigen Einheiten verwirklicht werden. Ein Spacer entkoppelt dabei die molekulare Beweglichkeit der nichtlinear-optischen Einheiten von der Polymerkette; gleichzeitig nimmt jedoch die Glasübergangstemperatur drastisch ab. Damit ist aber bei Gebrauchstemperaturen im Bereich der Glasübergangstemperatur der Polymeren der Verlust der molekularen Orientierung der nichtlinear-optischen Moleküleinheiten und der Verlust der nichtlinear-optischen Aktivität zu erwarten.

Aufgabe der Erfindung ist es, das Angebot an Polymeren für nichtlinear-optische Medien zu erweitern und insbesondere Polymere zur Verfügung zu stellen, die eine technisch ausreichende Glasübergangstemperatur aufweisen.

Dies wird erfindungsgemäß durch Epoxidharze folgender Struktur erreicht:

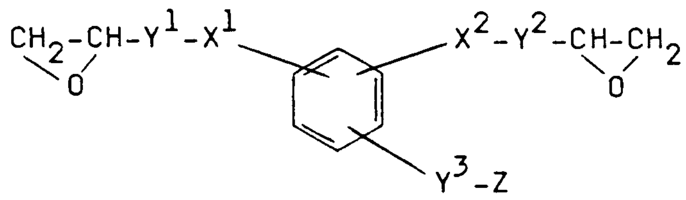 ,

wobei folgendes gilt:

$X^1$ und $x^2$ = O, S, COO oder OOC;
$Y^1$ und $Y^2$ = Alkylen (linear oder verzweigt) mit 1 bis 3 C-Atomen;
$Y^3$ = Alkylen (linear oder verzweigt) mit 2 bis 20 C-Atomen, wobei eine oder mehrere, nicht benachbarte $CH_2$-Gruppen, mit Ausnahme der bindenden $CH_2$-Gruppe zum Rest Z, durch O, S oder NR (R = H oder $C_1$- bis $C_6$-Alkyl) ersetzt sein können;
Z ist ein mit einem Elektronendonator (D) und einem Elektronenakzeptor (A) substituiertes konjugiertes $\pi$-Elektronensystem (E) der Struktur -D-E-A, wobei folgendes gilt:

E = 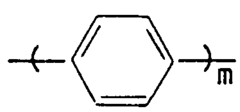 , 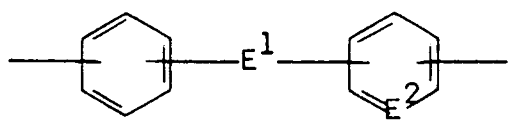 oder

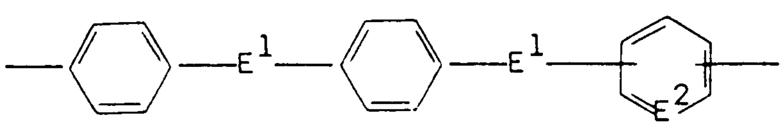 ,

mit
m = 1 bis 3,
$E^1$ = $-(CH=CH)_n-$, -N=N-, -CH=N-, -N=CH- oder -C≡C-, mit n = 1 bis 3, und
$E^2$ = CH oder N;
D = O, S, $NR^1$, $PR^2$ oder $NR^3$-$NR^4$, mit $R^1$ $R^2$ $R^3$ und $R^4$ = Wasserstoff, Alkyl, Alkenyl, Phenyl; und
A = Halogen, NO, $NO_2$, CN, $CF_3$, $COR^5$, $COOR^6$, $SO_2OR^7$, $SO_2NR^8_2$,

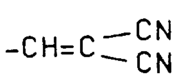 oder 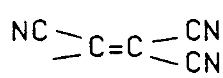 ,

mit $R^5$, $R^6$, $R^7$ und $R^8$ = Wasserstoff, Alkyl, Alkenyl, Phenyl.

Vorzugsweise gilt folgendes:

$X^1$ = $X^2$ = O oder COO,
$Y^1$ = $Y^2$ = $CH_2$,
$Y^3$ = $O-(CH_2)_o$, mit o = 2 bis 6, und
Z = -D-E-A mit D = O oder $NR^1$, A = $NO_2$ oder

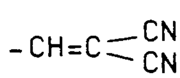 ,

und

E = 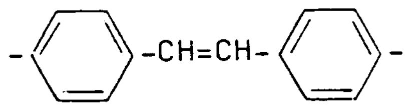 oder 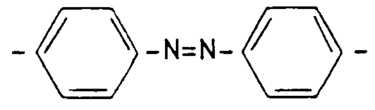 .

Die konjugierten $\pi$-Elektronensysteme (E) können auf der Elektronendonatorseite - zusätzlich zu D - mit wenigstens einem weiteren Elektronendonator und auf der Elektronenakzeptorseite - zusätzlich zu A - mit wenigstens einem weiteren Elektronenakzeptor substituiert sein. Diese Substituenten sollten so ausgewählt werden, daß die Summe der Hammetkonstanten $\sigma$ der jeweils zusätzlichen Substituenten den Wert des vorhandenen Substituenten (D bzw. A) nicht überschreitet.

Die Erfindung besteht somit in neuen Epoxidharzen der vorstehend genannten Art. Ferner besteht die Erfindung in der Verwendung dieser Epoxidharze in vernetzter Form für nichtlinear-optische Medien bzw. in nichtlinear-optischen Polymeren in Form vernetzter Epoxidharze der vorstehenden Struktur.

Es wurde nämlich überraschenderweise gefunden, daß vernetzte Epoxidharze der beschriebenen Art mit kovalent gebundenen nichtlinear-optischen Moleküleinheiten einerseits die vorstehend genannten Nachteile nicht aufweisen, andererseits aber die bekannt guten polymerspezifischen Eigenschaften besitzen, wie Verarbeitbarkeit zu dünnen Schichten im $\mu$m-Bereich, hohe Konzentration an nichtlinear-optischen Moleküleinheiten, niedrige optische Dämpfung und technisch ausreichende Glasübergangstemperatur. Dies liegt darin begründet, daß bei den erfindungsgemäßen Epoxidharzen durch Vernetzung eine Strukturierung der nichtlinear-optischen Polymerschicht zu Wellenleiterstrukturen erfolgen kann, wie sie bei unvernetzten Polymeren nicht möglich sind.

Die Herstellung nichtlinear-optischer Polymerer durch Vernetzung auf chemischem Weg ist bereits bekannt (siehe dazu: "J. Appl. Phys.", Vol. 66 (1989), Seiten 3241 bis 3247). Dazu werden zunächst durch Umsetzung von Bisphenol A-diglycidylether mit 4-Nitro-1.2-phenylendiamin lösliche Prepolymere hergestellt, die dann durch Erhitzen in unlösliche vernetzte Polymere übergeführt werden. Auf entsprechende Weise können auch nichtlinear-optische Polymere aus N,N-Diglycidyl-4-nitroanilin und N-(2-Aminophenyl)-4-nitroanilin hergestellt werden (siehe dazu: "Appl. Phys. Lett.", Vol. 56 (1990), Seiten 2610 bis 2612). Aus "J. Opt. Soc. Am. B", Vol. 7 (1990), Seiten 1239 bis 1250, sind ferner elektrooptische Polymerfilme bekannt, die durch Umsetzung von 4-Nitroanilin mit Bisphenol A-diglycidylether erhalten werden.

Die Herstellung der Epoxidharze nach der Erfindung sowie die Synthese der Vorstufen erfolgt nach an sich bekannten Verfahren (vgl. dazu die Ausführungsbeispiele). Die Vernetzung der Epoxidharze kann entweder thermisch oder photochemisch erfolgen.

Bei der thermischen Vernetzung werden den Epoxidharzen nach der Erfindung - in einem entsprechenden molaren Verhältnis - vernetzungswirksame Reagenzien zugesetzt, im allgemeinen Verbindungen mit aciden Wasserstoffatomen, Substanzen, aus denen derartige Verbindungen erzeugt werden können, oder Verbindungen mit elektrophilen Gruppierungen. Die Vernetzung erfolgt dann bei erhöhter Temperatur, vorzugsweise bei einer Temperatur, die 15°C oberhalb der Glasübergangstemperatur des vernetzten Endproduktes, d.h. des Polymeren, liegt. Gegebenenfalls kann die Vernetzungsreaktion durch Zugabe eines Beschleunigers katalysiert werden. Die zur thermischen Vernetzung eingesetzten Verbindungen bzw. Substanzen sind an sich bekannt. Vorzugsweise dienen dazu Carbonsäureanhydride, Phenole und aliphatische, cycloaliphatische oder aromatische Amine.

Geeignete Carbonsäureanhydride sind insbesondere Phthalsäureanhydrid, Tetrahydro-, Hexahydro-, Methyltetrahydro- und Endomethylentetrahydrophthalsäureanhydrid, Pyromellithsäure-, Trimellithsäure und Benzophenontetracarbonsäureanhydrid sowie Maleinsäureanhydrid/Styrol-Copolymere. Als Amine dienen insbesondere 4.4'-Diaminodiphenylmethan sowie dessen o,o'-alkylsubstituierte Derivate und hydriertes 4.4'-Diaminodiphenylmethan, 4.4'-Diaminodiphenylether, 4.4'-Diaminodiphenylsulfon, 2.4-Diamino-3.5-diethyltoluol, Isophorondiamin, Diethylentriamin, Triethylentetramin und Polyaminoamide auf der Basis von Diethylentriamin.

Die photochemische Vernetzung der Epoxidharze nach der Erfindung erfolgt durch Licht kürzerer Wellenlänge, vorzugsweise durch Licht im UV-Bereich (290 bis 390 nm). Zur Auslösung der photochemischen Vernetzung werden dabei Initiatoren zugesetzt, die unter dem Einfluß von Licht Lewis- oder Brönstedsäuren freisetzen; derartige Verbindungen sind an sich bekannt. Vorzugsweise dienen als Initiatoren Aryldiazonium-, Diaryljodonium- oder Triarylsulfoniumsalze, die als Anion Tetrafluoroborat, Hexafluorophosphat oder Hexafluoroantimonat aufweisen, sowie Aren-Eisen-Salze.

Zur Verbesserung der Oberflächenbeschaffenheit, der Verarbeitbarkeit und/oder der Verträglichkeit mit Polymeren können den Epoxidharzen - je nach Einsatzzweck - Verarbeitungshilfsmittel zugesetzt werden.

Dies sind beispielsweise Thixotropiermittel, Verlaufmittel, Weichmacher, Netzmittel, Gleitmittel und Bindemittel.

Die Epoxidharze nach der Erfindung werden in gelöster oder flüssiger Form, gegebenenfalls zusammen mit vernetzungswirksamen Verbindungen oder Initiatoren, durch Aufschleudern, Tauchen, Bedrucken oder Bestreichen auf ein Substrat aufgebracht. Auf diese Weise erhält man eine nichtlinear-optische Anordnung, wobei die Epoxidharze oder entsprechende Prepolymere vor und/oder nach der Vernetzung in elektrischen Feldern dipolar ausgerichtet werden. Nach dem Abkühlen werden Polymermaterialien mit ausgezeichneten nichtlinear-optischen Eigenschaften und - bedingt durch die Vernetzung - erhöhter Orientierungsstabilität und somit erhöhter Langzeitstabilität, auch bei erhöhten Gebrauchstemperaturen, erhalten.

Zur Erzeugung der nichtlinear-optischen Medien werden besonders vorteilhaft oligomere niedermolekulare Prepolymere der erfindungsgemäßen Epoxidharze verwendet. Die Herstellung dieser Prepolymeren erfolgt in an sich bekannter Weise, wobei die Epoxidharze mit einem Unterschuß an der vernetzungswirksamen Verbindung zur Reaktion gebracht werden. Nach dem Aufbringen auf ein Substrat werden die Prepolymeren - oberhalb der Glasübergangstemperatur - dipolar ausgerichtet und anschließend zu den nichtlinear-optischen Polymeren (mit verbessertem Eigenschaftsprofil) vernetzt.

Anhand von Ausführungsbeispielen soll die Erfindung noch näher erläutert werden.

Beispiel 1

Herstellung von 4-Hydroxy-4'-nitrostilben

72,4 g p-Nitrophenylessigsäure und 48,8 g p-Hydroxybenzaldehyd werden zusammen mit 60 ml Piperidin unter Rühren auf 140 °C erhitzt. Nach beendeter Reaktion wird die erkaltete Masse aus Eisessig umkristallisiert (Schmp.: 207 °C); Ausbeute: 70 %.

Beispiel 2

Herstellung von 4-(6-Bromhexyloxy)4'-nitrostilben

120,6 g 4-Hydroxy-4'-nitrostilben (siehe Beispiel 1), 153,8 ml 1.6-Dibromhexan und 96,5 g Kaliumcarbonat werden zusammen mit einer geringen Menge Kaliumjodid 48 h in absolutem Aceton unter Rückfluß erhitzt. Nach dem Abkühlen werden die anorganischen Salze abfiltriert, das Lösungsmittel wird im Vakuum entfernt und der Rückstand aus Ethanol umkristallisiert (Schmp.: 101 °C); Ausbeute: 60 %.

Beispiel 3

Herstellung von 5-[4-(6-Oxyhexyloxy)-4'-nitrostilben]-isophthalsäuredimethylester
60,6 g 4-(6-Bromhexyloxy)-4'-nitrostilben (siehe Beispiel 2),
37,8 g 5-Hydroxyisophthalsäuredimethylester und 29 g Kaliumcarbonat werden zusammen mit einer geringen Menge Kaliumjodid 72 h in absolutem Aceton unter Rückfluß erhitzt. Der ausgefallene Feststoff wird heiß abfiltriert, in Methylenchlorid aufgenommen und an Kieselgel chromatographiert (Schmp.: 135 °C); Ausbeute: 80 %.

Beispiel 4

Herstellung von 5-[4-(6-Oxyhexyloxy)-4'-nitrostilben]-isophthalsäure

10 g 5-[4-(6-Oxyhexyloxy)-4'-nitrostilben]-isophthalsäuredimethylester (siehe Beispiel 3) und 150 ml 10 %ige Natronlauge werden zusammen 3 h unter Rückfluß erhitzt. Nach dem Abkühlen wird die Reaktionslösung mit konzentrierter Salzsäure auf einen pH-Wert von 1 bis 2 eingestellt, der Niederschlag wird abfiltriert, mit Wasser neutral gewaschen, getrocknet und aus Ethanol umkristallisiert (Schmp.: 210 °C) ; Ausbeute: 96 %.

Beispiel 5

Herstellung von 5-[4-(6-Oxyhexyloxy)-4'-nitrostilben]-isophthalsäurediglycidylester

10,9 g 5-[4-(6-Oxyhexyloxy)-4'-nitrostilben]-isophthalsäure (siehe Beispiel 4) werden in 40 g Epichlorhydrin suspendiert, mit 43,2 mg Benzyltriethylammoniumchlorid versetzt und 4 h bei 105 °C gerührt. Anschließend wird unter Rückfluß erhitzt, und dann werden 3 ml 50 %ige Natronlauge in der Weise zugetropft, daß Wasser kontinuierlich azeotrop aus dem Reaktionsgemisch entfernt wird. Nach beendeter Zugabe der Natronlauge wird noch 15 min erhitzt, um die letzten Reste an Wasser zu entfernen. Nach dem Abkühlen wird die Reaktionsmischung mit Ethylacetat versetzt, der ausgefallene Rückstand wird abfiltriert und aus Toluol umkristallisiert (Schmp.: 160 °C); Ausbeute: 85 %.

Beispiel 6

Zur Vernetzung der glycidylfunktionalisierten nichtlinear-optischen Moleküleinheiten wird der Isophthalsäureester nach Beispiel 5 - unter Zusatz von 1 Mol-% N,N'-Dimethylbenzylamin - mit Carbonsäureanhydriden vermischt und bei erhöhter Temperatur ausgehärtet. Die dabei erhaltenen Ergebnisse sind in Tabelle 1 zusammengefaßt ($T_G$ = Glasübergangstemperatur), wobei folgendes gilt:

EP = 5-[4-(6-Oxyhexyloxy)-4'-nitrostilben]-isophthalsäurediglycidylester
BSA = Bernsteinsäureanhydrid
HHPSA = Hexahydrophthalsäureanhydrid
PSA = Phthalsäureanhydrid
PMSA = Pyromellithsäureanhydrid

Tabelle 1

| Polymer Nr. | Zusammensetzung in Mol-% | Härtungstemperatur in °C | $T_G$ in °C |
|---|---|---|---|
| 1 | 100 EP:100 BSA | 140 | 110 |
| 2 | 100 EP:100 HHPSA | 140 | 124 |
| 3 | 100 EP:100 PSA | 140 | 127 |
| 4 | 100 EP: 50 PMSA | 150 | 137 |

Beispiel 7

Für die elektrooptischen Untersuchungen werden die erfindungsgemäßen Epoxidharze oder entsprechende Prepolymere, gegebenenfalls zusammen mit vernetzungswirksamen Verbindungen, in einem geeigneten Lösungsmittel auf ITO-beschichtetes Glas (ITO = Indium-Zinn-Oxid) durch Spin-coating aufgebracht; die Schichtdicke von in dieser Weise hergestellten Filmen beträgt üblicherweise 3 bis 6 µm. Für das elektrische Polen, zur Erzielung einer hohen nicht-zentrosymmetrischen Orientierung, wird auf den Film (aus dem Epoxidharz) eine Goldelektrode aufgesputtert; die Gegenelektrode ist dabei die lichtdurchlässige ITO-Schicht. Nach dem Erwärmen der Probe bis in den Glasübergangstemperaturbereich wird eine Gleichspannung angelegt, wobei die erforderliche Spannungserhöhung auf das Orientierungsverhalten der nichtlinear-optischen Moleküleinheiten abgestimmt wird, um elektrische Durchschläge und somit eine Zerstörung des Films zu vermeiden. Nach Erreichen einer Polungsfeldstärke von 50 bis 100 V/µm genügt eine Polungsdauer von 15 min zur Orientierung der nichtlinear-optischen Moleküleinheiten. Anschließend wird die Probe vernetzt, und zwar thermisch (entsprechend Beispiel 6) oder photochemisch, und dann wird die Probe - mit konstant anliegendem elektrischen Feld - bis auf Raumtemperatur abgekühlt, womit die Orientierung fixiert wird.

Die elektrooptische Untersuchung der Polymerproben erfolgt durch interferometrische Messung eines schräg eingestrahlten Laserstrahls nach Einfachreflexion an der Goldelektrode. Der dazu erforderliche Meßaufbau und die Meßauswertung sind bekannt (siehe beispielsweise: "Appl. Phys. Lett.", Vol. 56 (1990), Seiten 1734 bis 1736). Die elektrooptischen Koeffizienten $r_{33}$ der Polymeren nach Beispiel 6, d.h. der vernetzten Epoxidharze, die auf eine Polungsfeldstärke von 70 V/µm bezogen sind, sind in Tabelle 2 zusammengefaßt.

Tabelle 2

| Polymer Nr. | elektrooptischer Koeffizient in pm/V |
|---|---|
| 1 | 4,2 |
| 2 | 3,2 |
| 3 | 3,4 |
| 4 | 3,5 |

## Patentansprüche

**1.** Epoxidharze der Struktur

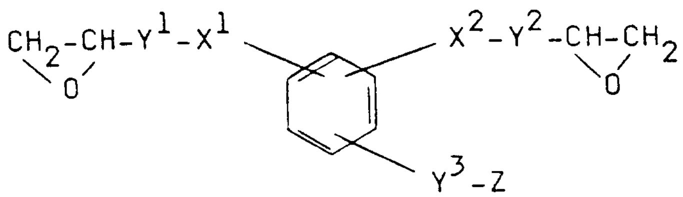

,

wobei folgendes gilt:

| | |
|---|---|
| $X^1$ und $X^2$ | = O, S, COO oder OOC; |
| $Y^1$ und $Y^2$ | = Alkylen (linear oder verzweigt) mit 1 bis 3 C-Atomen; |
| $Y^3$ | = Alkylen (linear oder verzweigt) mit 2 bis 20 C-Atomen, wobei eine oder mehrere, nicht benachbarte $CH_2$-Gruppen, mit Ausnahme der bindenden $CH_2$-Gruppe zum Rest Z, durch O, S oder NR (R = H oder $C_1$- bis $C_6$-Alkyl) ersetzt sein können; |
| Z | ist ein mit einem Elektronendonator (D) und einem Elektronenakzeptor (A) substituiertes konjugiertes $\pi$-Elektronensystem (E) der Struktur -D-E-A, wobei folgendes gilt: |

E = 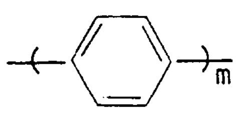 , 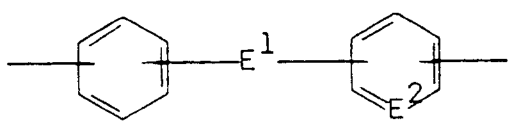 oder

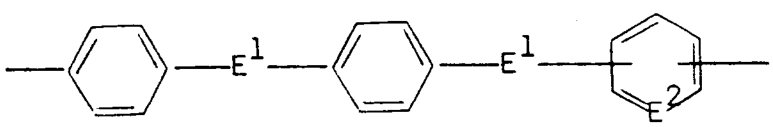 ,

mit

| | |
|---|---|
| m | = 1 bis 3, |
| $E^1$ | = $-(CH=CH)_n-$, $-N=N-$, $-CH=N-$, $-N=CH-$ oder $-C\equiv C-$, mit n = 1 bis 3, und |
| $E^2$ | = CH oder N; |
| D | = O, S, $NR^1$, $PR^2$ oder $NR^3$-$NR^4$, mit |
| $R^1$, $R^2$, $R^3$ und $R^4$ | = Wasserstoff, Alkyl, Alkenyl, Phenyl; und |
| A | = Halogen, NO, $NO_2$, CN, $CF_3$, $COR^5$, $COOR^6$, $SO_2OR^7$, $SO_2NR^8_2$ , |

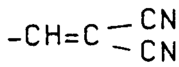 oder 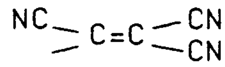 ,

mit $R^5$, $R^6$, $R^7$ und $R^8$ = Wasserstoff, Alkyl, Alkenyl, Phenyl.

2. Epoxidharze nach Anspruch 1, **dadurch gekennzeichnet,** daß folgendes gilt:
   - $X^1$ = $X^2$ = O oder COO,
   - $Y^1$ = $Y^2$ = $CH_2$,
   - $Y^3$ = O-$(CH_2)_o$, mit o = 2 bis 6, und
   - Z = -D-E-A mit D = O oder $NR^1$, A = $NO_2$ oder

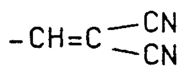 ,

und

E = 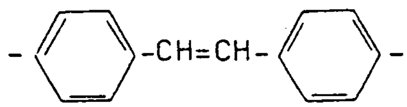 oder 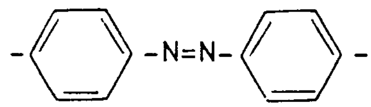 .

3. Verwendung der Epoxidharze nach Anspruch 1 oder 2 in vernetzter Form für nichtlinear-optische Medien.

**Claims**

1. Epoxy resins with the structure

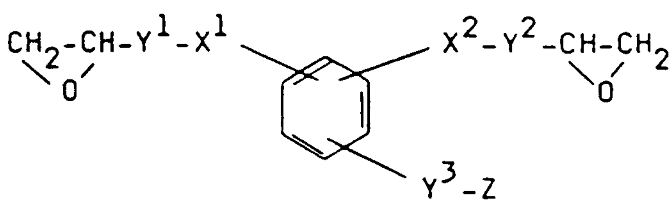 ,

where the following applies:
   - $X^1$ and $X^2$ = O, S, COO or OOC;
   - $Y^1$ and $Y^2$ = alkylene (linear or branched) with 1 to 3 C atoms;
   - $Y^3$ = alkylene (linear or branched) with 2 to 20 C atoms, where one or more non-adjacent $CH_2$ groups, with the exception of the binding $CH_2$ group to the residue Z, can be replaced with O, S or NR (R = H or $C_1$ to $C_6$ alkyl);

Z is a conjugated $\pi$ electron system (E) with the structure -D-E-A, substituted with an electron donor (D) and an electron acceptor (A), where the following applies:

E = 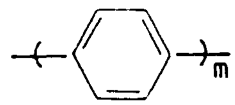 , 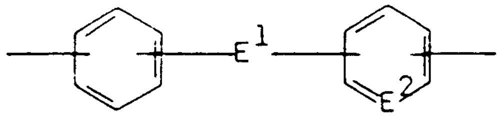

or

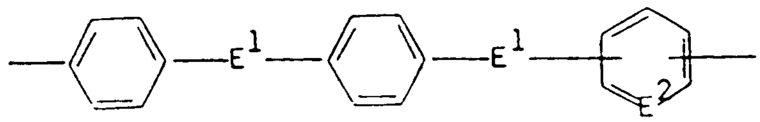 ,

with

m = 1 to 3,

$E^1$ = $-(CH=CH)_n-$, $-N=N-$, $-CH=N-$, $-N=CH-$ or $-C\equiv C-$, with n = 1 to 3, and

$E^2$ = CH or N;

D = O, S, $NR^1$, $PR^2$ or $NR^3$-$NR^4$,

with $R^1$, $R^2$, $R^3$ and $R^4$ = hydrogen, alkyl, alkenyl, phenyl; and

A = halogen, NO, $NO_2$, CN, $CF_3$, $COR^5$, $COOR^6$, $SO_2OR^7$, $SO_2NR_2^8$,

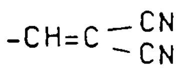 or 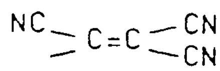 ,

with $R^5$, $R^6$, $R^7$ and $R^8$ = hydrogen, alkyl, alkenyl, phenyl.

**2.** Epoxy resins according to claim 1,
characterised in that the following applies:

$X^1$ = $X^2$ = O or COO,

$Y^1$ = $Y^2$ = $CH_2$

$Y^3$ = $O-(CH_2)_o$, with o = 2 to 6, and

Z = -D-E-A with D = O or $NR^1$, A = $NO_2$ or

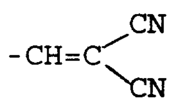 ,

and

E = 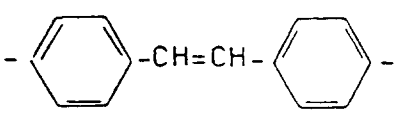 or 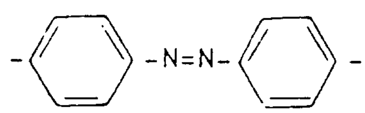 .

**3.** Use of the epoxy resins according to claim 1 or 2 in cross-linked form for non-linear optical media.

**Revendications**

**1.** Epoxy-résines de structure

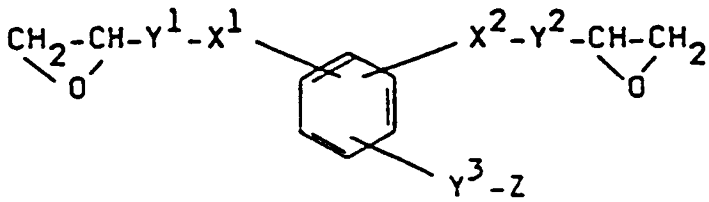

,

dans laquelle :

| | |
|---|---|
| $X^1$ et $X^2$ | représentent O, S, COO ou OOC; |
| $Y^1$ et $Y^2$ | représentent un groupe alkylène (linéaire ou ramifié) contenant de 1 à 3 atomes de carbone; |
| $Y^3$ | représente un groupe alkylène (linéaire ou ramifié) contenant de 2 à 20 atomes de carbone, dans lequel un ou plusieurs groupes $CH_2$ non voisins, à l'exception du groupe de liaison $CH_2$ par rapport au radical Z, peuvent être remplacés par O, S ou NR (R = H ou un groupe alkyle en $C_1$-$C_6$); |
| Z | représente un système d'électrons $\pi$ conjugué (E) substitué par un donneur d'électrons (D) et par un accepteur d'électrons (A), de structure -D-E-A, |

E = 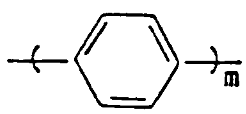 , 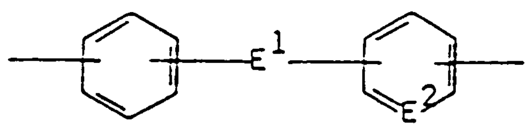 ou

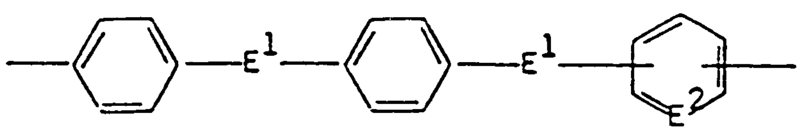 ,

dans laquelle

| | |
|---|---|
| m | représente 1 - 3, |
| $E^1$ | représente $-(CH=CH)_n-$, -N=N-, -CH=N-, -N=CH-ou -C≡C-, n représentant 1 à 3, et |
| $E^2$ | représente CH ou N; |
| D | représente 0, S, $NR^1$, $PR^2$ ou $NR^3$-$NR^4$, où $R^1$, $R^2$, $R^3$ et $R^4$ représentent un atome d'hydrogène, un groupe alkyle, un groupe alcényle, un groupe phényle, et |
| A | représente un atome d'halogène, NO, $NO_2$, CN, $CF_3$, $COR^5$, $COOR^6$, $SO_2OR^7$, $SO_2NR^8{}_2$, |

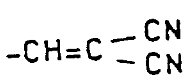 ou 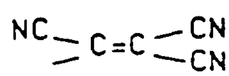 ,

où $R^5$, $R^6$, $R^7$ et $R^8$ représentent un atome d'hydrogène, un groupe alkyle, un groupe alcényle, un groupe phényle.

**2.** Epoxy-résines selon la revendication 1, caractérisées en ce que :

| | |
|---|---|
| $X^1$ | = $X^2$ = O ou COO; |
| $Y^1$ | = $Y^2$ = $CH_2$ |
| $Y^3$ | = $O-(CH_2)_o$ où o = 2 à 6; et |
| Z | = -D-E-A où D = O ou $NR^1$, A = $NO_2$ ou |

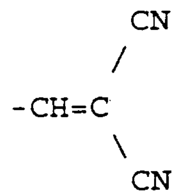 ,

et

E = 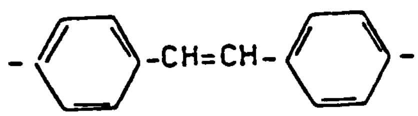 ou 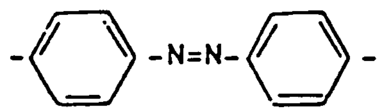 .

3. Utilisation des époxy-résines selon la revendication 1 ou 2, sous forme réticulée pour des milieux optiques non linéaires.